# DEMANDE DE BREVET EUROPEEN

(11) **EP 1 170 382 A2**
(43) Date de publication de la demande: **09.01.2002**
(21) Numéro de dépôt: 01401823.8
(22) Date de dépôt: 06.07.2001
(51) Int. Cl.: C12Q 1/68, C12N 1/20, C12N 15/31

(54) **Méthode de diagnostic d'une infection bactérienne chez les naissains d'huîtres Crassostrea gigas**

(30) Priorité: 06.07.2000 FR 0008830
(71) Demandeur: Institut Francais de Recherche pour l'Exploitation de la Mere(Ifremer), 92130 Issy-les-Moulineaux Cedex (FR); GRAINOCEAN, F-17410 Saint Martin de Re (FR)
(72) Inventeur: Marissal, Eric, 17410 Saint Martin de re (FR); Berthe, Franck, 17300 Rochefort (FR); Waechter, Magali, 17350 Saint Savinien (FR)
(74) Mandataire: Le Guen, Gérard

(57) **Abrégé**

L'invention concerne une méthode *in vitro* de diagnostic d'une infection bactérienne atteignant les naissains d'huîtres creuses, Crassostrea *gigas,* dans laquelle on détecte la présence dans ces naissains d'une souche bactérienne de *Vibrio splendidus II,* par analyse d'un échantillon de tissus de naissains.

## Description

L'invention a trait à une méthode de diagnostic d'une infection bactérienne à *Vibrio splendidus II* chez des naissains d'huîtres de l'espèce *Crassostrea gigas,* et à une nouvelle souche bactérienne de l'espèce *Vibrio splendidus II* plus particulièrement pathogène pour ces huîtres.

La production d'huître creuse, *Crassostrea gigas*, est la première production française de coquillages. Cette production se place au premier rang européen, et au 5^{ème} rang mondial. Le naissain est principalement obtenu par captage naturel mais l'utilisation de naissain d'écloserie est en progression depuis quelques années. A la faveur de températures élevées, de forts taux de mortalité de larves et de naissains d'huître creuse, *Crassostrea gigas*, (60 à 100%) sont rapportés régulièrement depuis 1991, dans les écloseries et les nurseries, ainsi qu'en milieu naturel. La mortalité atteignant *Crassostrea gigas* n'est pas identique à la Juvenile Oyster Disease (JOD) de l'huître américaine *Crassostrea virginica*, où l'on observe une croissance différentielle des deux valves ainsi que des dépôts de conchioline sur la face interne des valves pouvant former des anneaux bruns, symptomatiques de la JOD.

Des études menées dans le but d'identifier les causes de ces épisodes de mortalité de *C. gigas* ont révélé la présence de différents agents infectieux. Des rickettsies ont été décrites, associées aux épisodes de mortalité, sans que leur responsabilité ait été démontrée (Renault et al, 1995). Un virus de type Herpès a ainsi été mis en évidence par observations en microscopie électronique à transmission (Nicolas *et al*., 1992) en relation avec des épisodes de mortalité sur des larves d'huître creuse. La pathogénicité de ce virus de type Herpès pour l'huître creuse a ensuite été prouvée expérimentalement pour les stades larvaires (Le Deuff *et al*., 1994) mais n'a pas été démontrée pour le naissain, touché lui aussi par ce phénomène de mortalité.

Il existait donc un besoin d'identifier le ou les agent(s) pathogène(s) des naissains d'huîtres *Crassostrea gigas.*

Les auteurs de la présente invention ont maintenant mis en évidence l'implication de bactéries de l'espèce *Vibrio splendidus II* dans les épisodes de mortalité atteignant ces naissains d'huîtres.

A partir de ces résultats, ils proposent une méthode *in vitro* de diagnostic d'une infection bactérienne des naissains d'huîtres *Crassostrea gigas*, dans laquelle la présence dans ces naissains d'huîtres d'une souche de *Vibrio splendidus II* est détectée, par analyse d'un échantillon de tissus de naissains d'huître, par exemple d'un broyat de chair.

Cette détection peut être par exemple réalisée par ribotypage des souches bactériennes préalablement isolées des naissains. Ce ribotypage peut être effectué par restriction des ADN génomiques de ces souches bactériennes puis hybridation à l'aide de sondes 16/23S après transfert de Southern (Grimont et al., 1995).

Par "naissain d'huître", on entend une jeune huître après la métamorphose, qui se caractérise par la fixation de larves après une période de dix à vingt jours d'élevage larvaire, et jusqu'à l'âge d'un an environ.

Les auteurs de l'invention ont plus particulièrement identifié une nouvelle souche bactérienne, désignée S6, qui s'avère extrêmement pathogène pour les naissains d'huître creuse, *Crassostrea gigas*. Cette souche a été déposée le 24 février 2000 à la Collection Nationale de Cultures de Microorganismes (CNCM) de l'Institut Pasteur, sous les termes du Traité de Budapest et a reçu le numéro d'enregistrement 1-2385.

L'analyse des séquences de l'ADN ribosomal 16S de cette souche a permis de montrer qu'il s'agissait d'une nouvelle souche de *Vibrio splendidus II*.

La séquence obtenue, qui code pour la petite sous-unité ribosomale, est présentée dans le listage de séquences annexé (SEQ ID n° 1). Un acide nucléique comprenant la séquence SEQ ID n° 1 fait également partie de l'invention.

Cet acide nucléique peut être notamment utile pour la fabrication de sondes nucléiques pour la détection de *Vibrio splendidus II*.

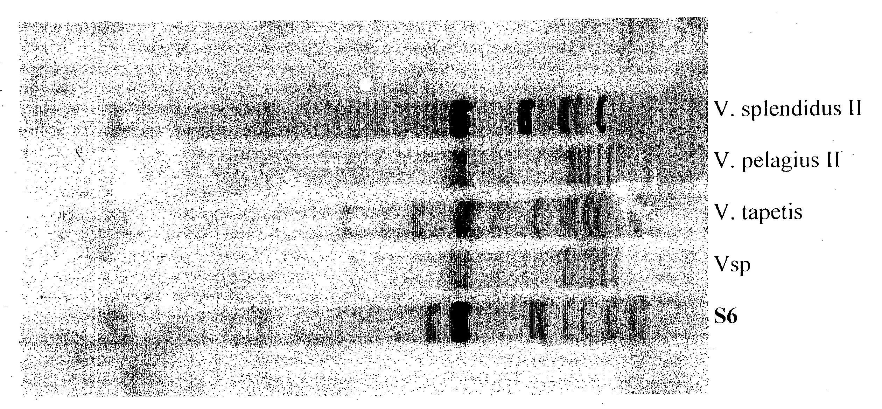

La figure et les exemples ci-après illustrent l'invention sans en limiter sa portée.

### LEGENDE DE LA FIGURE :

La figure en annexe représente un profil de restriction des gènes ribosomaux (ribotype) de différentes souches de *Vibrio*.

### EXEMPLES

### EXEMPLE 1 : Recherche de souches bactériennes associées à des épisodes de mortalité

### 1.1. Matériels et méthodes :

### 1.1.1. Prélèvements :

Les prélèvements sont effectués sur le site de l'écloserie, de la nurserie et sur filières en mer à chaque étape de la production de naissain :

**Tableau 1 :**

| **Echantillonnage** | | |
|---|---|---|
| **Etapes de l'élevage** | **Prélèvements** | **nombre de prélèvements** |
| **Fécondation** | eau de fécondation des géniteurs | 1 |
| **Elevage larvaire** | larves et eau d'élevage larvaire | 1/48h |
| **Fixation** | naissain | 1/semaine |
| **Nurserie** | naissain | 1/semaine |
| **Filière en mer** | naissain | 1/mois |

Le suivi d'élevage est effectué systématiquement sur chaque nouveau lot d'élevage. Les souches isolées sont conservées et permettent de rechercher la présence d'une souche pathogène sur un lot si une mortalité se déclare au cours de l'élevage.

Le suivi du cheptel permet aussi d'observer un ensemble de signes et paramètres de milieu concomitants à l'apparition d'un épisode de mortalité.

La recherche de souches pathogènes est effectuée sur un échantillon particulier prélevé lors d'un épisode de mortalité (~60 %) survenu en septembre 1997 sur du naissain de captage naturel placé en prégrossissement sur des filières en mer.

D'autres prélèvements ont été effectués sur le même lot :
- avant mortalité en juin 1997
- à 10 % de mortalité en juillet 1997
- à 25 % de mortalité en juillet 1997
- à 0 % de mortalité en septembre (lot qui présentera par la suite une mortalité de 90%)
- à 30 % de mortalité en septembre 1997.

### 1.1.2. Identification des souches bactériennes :

### - Milieux de culture

Les souches bactériennes sont obtenues à partir de broyats de chair dilués en eau de mer stérile et étalés sur deux types de milieux : le milieu de Zobell (Zobell, 1956), permettant d'isoler la flore hétérotrophe totale et le TCBS, un milieu sélectif permettant d'isoler les souches de la flore vibrionaceae (Kobayashi *et al*, 1963).

### - Tests biochimiques d'identification de genre et d'espèce

La caractérisation des souches isolées en terme de genre et d'espèce est réalisée à partir de 35 tests biochimiques.

Ces tests sont : Gram, mobilité, oxydase, MEVAG, V.F., gélatinase, Citrate de Simmons, Kligler-Hajna, ADH, LDC, ODC, Indole, Voges-Proskauer, Nitrate, ONPG, croissance à 0, 6, 8, 10% de salinité, croissance à 4, 20, 35 et 40 °C... (Manual of methods for general bacteriology (1981). Gerhardt, Murray, Costilow, Nester, Wood, Krieg and Phillips editors. American Society for Microbiology, Washington).

Ces tests sont adaptés pour l'étude de souches marines (addition de NaCI à 2% ou d'eau de mer artificielle dans les milieux et réactifs). Les résultats de ces tests sont utilisés dans des arbres dichotomiques d'identification, construits à partir de données bibliographiques (Alsina *et al*, 1994).

### - Tests biochimiques complémentaires

### * Auxanogramme

L'auxanogramme utilisé pour cette étude est le « Biotype 100 » (BioMérieux, La Balme les Grottes, France). Ce test se présente sous la forme d'une galerie de 100 cupules et met en évidence l'utilisation de certains composés carbonés comme unique source d'énergie en milieu minimum.

### *APIZYM

Le système API ZYM (BioMérieux référence 2 520 0) est une microméthode de recherche d'activités enzymatiques se composant d'une galerie de 10 cupules contenant les substrats de certaines enzymes. La présence de l'enzyme est révélée grâce à un réactif coloré qui réagit avec le produit de dégradation du substrat.

### 1. 2 Résultats :

Les tests nécessaires à la caractérisation des souches isolées en terme de genre et d'espèce ont permis de connaître la composition de la flore vibrionaceae du prélèvement effectué au moment de l'épisode de mortalité choisi pour la recherche de souches bactériennes pathogènes (60 % de taux de mortalité)

**Tableau 1 :**

| **Caractérisation du genre et de l'espèce** | |
|---|---|
| N° de la souche | Nom de genre et d'espèce |
| 1 | *Vibrio aestuarianus - Listonella anguillara* |
| 2 | *Vibrio mediterranei* |
| 3 | *Vibrio splendidus I - Vibrio tubiashii* |
| 4 | *Vibrio splendidus I - Vibrio tubiashii* |
| 5 | *Vibrio mediterranei* |
| 6 | *Vibrio splendidus II* |
| 7 | *Vibrio costicola* |
| 8 | *Vibrio splendidus II* |
| 9 | *Vibrio splendidus II* |

**Remarque :** Le test de luminescence est négatif pour la souche « S6 » (V. *splendidus I* : luminescence + ; *V. splendidus II* : luminescence -).

### EXEMPLE 2 : Modèle de pathologie expérimentale

### 2.1. Matériels et méthodes

Lemodèle de reproduction expérimentale de la mortalité s'appuie sur l'inoculation d'une souche bactérienne par balnéation.

Le témoin positif de l'expérience correspond à la souche de référence de *Vibrio tubiashii*^{*T*}; (CIP 102760) pathogène pour le naissain d'huître creuse. L'inoculation s'effectue par balnéation du naissain dans une solution concentrée (10⁷ à 10⁸ bactéries par ml) de bactéries.

Les souches bactériennes étudiées sont testées dans les mêmes conditions que la souche témoin positif.

La vérification du postulat de Koch s'effectue par réisolement des souches inoculées en pathologie expérimentale sur du naissain subissant un épisode de mortalité.

### 2.2. Résultats :

L'inoculation de la souche témoin positif (*Vibrio tubiashii*^{*T*}; CIP 102760) a entraîné une mortalité (taux cumulé de 90 %) après 55 jours. La souche a pu être isolée sur TCBS à partir de naissain mort, cette souche étant nettement majoritaire par rapport aux autres souches du genre *Vibrio* présentes dans le naissain. Le postulat de Koch a été vérifié pour cette souche, permettant ainsi de valider le modèle de pathologie expérimentale.

Parmi les souches de *Vibrio* isolées lors de l'épisode de mortalité (60%), une seule a effectivement induit une mortalité équivalente à la souche témoin positif. Cette souche est « S6 » identifiée comme un *Vibrio splendidus II* (Alsina *et al*, 1994). La souche a pu être réisolée sur TCBS à partir de naissain contaminé subissant une forte mortalité (90 %), cette souche étant nettement majoritaire par rapport aux autres souches du genre *Vibrio* présentes dans le naissain. La souche « S6 » est considérée comme une souche bactérienne pathogène pour le naissain d'huître creuse, *Crassostrea gigas*.

### EXEMPLE 3 : Caractérisation de la souche S6

### 3.1. Matériels et méthodes :

### 3.1.1. Séquençage de l'ADNr 16S :

Le séquençage des ADNr 16S (Ruimy *et al*, 1994) est effectué pour positionner les souches pathogènes d'un point de vue phylogénique. Le gène codant pour la petite sous unité ribosomale est amplifié par amplification en chaîne (PCR) à l'aide d'amorces universelles d'*E*. *coll*. Le fragment amplifié est ensuite cloné et séquencé. La séquence obtenue (SEQ ID n° 1) a été comparée sur BLAST 2.0 (MEDLINE, Internet) avec des séquences de nucléotides de la banque de données Genbank.

### 3.1.2. Ribotypage :

La technique de ribotypage est particulièrement intéressante pour la comparaison des souches bactériennes du genre *Vibrio* (Grimont *et al*, 1995).

La molécule d'ADN native est coupée par une enzyme de restriction (Mlul) en fragments de restriction. Ces fragments sont ensuite séparés par électrophorèse en gel d'agarose puis transférés sur membrane par Southern blot et révélés par hybridation à l'aide d'un mélange de sondes (Regnault B., Grimont F., Grimont P.A.D., 1997. Universal ribotyping method using a chemically labelled oligonucleotide probe mixture. Res. Microbiol., 148:649-659) marquées par le ' DIG Oligonucleotide tailing kit (Boerhinger Mannheim). Les fragments sont révélés par immunodétection avec le DIG Oligonucléotide detection kit (Boerhinger Mannheim).

Un ensemble de souches biochimiquement proches isolées de différents prélèvements (eau de fécondation, larves présentant un épisode de mortalité, naissain de captage naturel avant mortalité, naissain de captage naturel à différents taux de mortalité, naissain d'écloserie subissant une faible mortalité en nurserie... etc.) ont été comparées par le ribotypage.

### 3.2. Résultats :

### 3.2.1. Séquençage de l'ADNr 16S de la souche « S6 »

La séquence de l'ADNr16S de la souche pathogène « S6 » a été comparée avec le programme BLAST 2.0 (MEDLINE, http://www.ncbi.nim.nih.gov/BLAST/) à la banque de données de nucléotides répertoriés dans la Genbank (NCBI). La souche S6 est proche mais différente de *Vibrio splendidus I* (ATCC 33125) et de *Vibrio splendidus II* (ATCC 25914 et ATCC 33789).

### 3.2.2. Ribotypage

Le ribotypage effectué avec la souche « S6 » et les souches de référence de *Vibrio splendidus I* et *Vibrio splendidus II* ont permis de montrer que la souche « S6 » présente un profil différent de la souche de référence *de Vibrio splendidus II* (cf figure en annexe).

### REFERENCES BIBLIOGRAPHIQUES

- **Alsina M. and Blanch A. R.** (1994) A set of keys for biochemical identification of environmental *Vibrio* species. *J. App*. *Bact.* **76,** 79-85.
- **Alsina M. and Blanch A. R.** (1994) Improvement and update of a set keys for biochemical identification of *Vibrio* species. *J. App*. *Bact*. **77,** 719-721.
- **Gerhardt, Murray, Costilow, Nester, Wood, Krieg and Phillips editors** (1981) American Society for Microbiology, Washington Manual of methods for general bacteriology.
- **Grimont F. and Grimont P. A. D.** (1995) Determination of rRNA gene restriction patterns. In *Methods in molecular biology* (Edited by Howard J. and Whitcombe D. M.), p. 181. Humana Press Inc, Totowa.
- **Kobayashi T., Enomoto R., Sakazaki R. and Kuwahara S.** (1963) A new selective isolation medium for pathogenic *Vibrios* : TCBS agar. *Jap. J. Bacteriol*. **58,** 387-391.
- **Le Deuff R. M., Nicolas J. L., Renault T. and Cochennec N.** (1994) Experimental transmission of Herpes-like virus to axenic larvae of Pacific oyster, *Crassostrea gigas. Bull*. *Eur*. *Ass. Fish Pathol*. **142,** 69-72.
- **Nicolas J. L., Comps M. and Cochennec N.** (1992) Herpes-like virus infecting Pacific oyster larvae, *Crassostrea gigas*. *Bull. Eur*. *Ass. Fish Pathol.* **12**, 11-13.
- **Renault et al,** (1995) Herpes-like viruses associated with high mortality levels in larvae and spat of pacific oysters, Crassostrea-giga - a comparative-study, the thermal effects on virus detection in hatchery-reared larvae, reproduction of the disease in axenic larvae, Vet. Res. 26(5-6):539-543.
- **Ruimy et al,** (1994) Phylogenetic analysis and assessment of the genera Vibrio, Photobacterium Aeromonas and Plesiomonas deduced from small-subunit rRNA sequences, Int. J. Syst. Bact. 44(3):416-426.
- **Zobell C. E.** (1956) Studies on marine bacteria. I. The cultural requirements of heterotrophic aerobes. *Journal of Marine Research* 124, 43-75.

## Revendications

1. Méthode *in vitro* de diagnostic d'une infection bactérienne atteignant les naissains d'huîtres *Crassostrea gigas,* dans laquelle on détecte la présence dans ces naissains d'huîtres d'une souche de *Vibrio splendidus II*, par analyse d'un échantillon tissulaire.

2. Méthode selon la revendication 1, dans laquelle ladite souche bactérienne est une souche déposée le 24 février 2000 à la CNCM, sous le numéro I-2385.

3. Méthode selon la revendication 1 ou 2, dans laquelle le diagnostic desdites souches est réalisé par ribotypage des souches bactériennes préalablement isolées des naissains.

4. Méthode selon la revendication 2, dans laquelle ladite souche bactérienne I-2385 est détectée à l'aide d'une sonde nucléique obtenue à partir de la séquence SEQ ID n° 1.

5. Souche bactérienne pathogène pour les naissains d'huîtres *Crassostrea gigas*, déposée le 24 février 2000 à la CNCM sous la numéro I-2385.

6. Acide nucléique comprenant la séquence SEQ ID n° 1.

7. Utilisation d'un acide nucléique selon la revendication 6 pour la fabrication de sondes nucléiques pour la détection de *Vibrio splendidus II*.

8. Utilisation selon la revendication 7 dans laquelle le *Vibrio splendidus II* est la souche bactérienne 1-2385 déposée le 24 février 2000 à la CNCM.
